# EUROPEAN PATENT APPLICATION

(11) **EP 4 667 488 A1**
(43) Date of publication of application: **24.12.2025**
(21) Application number: 24850871.5
(22) Date of filing: 31.07.2024
(51) Int. Cl.: C07K 14/78, C12N 15/70, C07K 1/107, C12N 15/12, C12N 1/21

(54) **SELF-CROSSLINKING RECOMBINANT HUMANIZED COLLAGEN POLYMER BIOMATERIAL AND PREPARATION METHOD THEREFOR**

(30) Priority: 04.08.2023 CN 202310983181
(71) Applicant: Shanxi Jinbo Bio-Pharmaceutical Co., Ltd., Shanxi 030032 (CN)
(72) Inventor: ZHANG, Yongjian, Taiyuan, Shanxi 030032 (CN); LAN, Xiaobin, Taiyuan, Shanxi 030032 (CN); WANG, Lingling, Taiyuan, Shanxi 030032 (CN); YANG, Xia, Taiyuan, Shanxi 030032 (CN)
(74) Representative: Bayramoglu et al.
(86) International application number: PCT/CN2024/108743
(87) International publication number: WO 2025/031225

(57) **Abstract**

Provided are a self-crosslinking recombinant humanized collagen polymer biomaterial and a preparation method therefor. Provided is a collagen self-assembled element, including (i) at least 21 consecutive amino acids at the C-terminus of the amino acid sequence as shown in SEQ ID NO. 1; or an amino acid sequence that is obtained by means of substitution, deletion or addition of one or more amino acids in the amino acid sequence as described in (i) and that retains the function of promoting the self-crosslinking of recombinant type III humanized collagen to form a triple helix structure; or an amino acid sequence that has at least 90%, 92%, 95%, 96%, 97%, 98% or 99% identity to the amino acid sequence as described in (i) and that retains the function of promoting the self-crosslinking of recombinant type III humanized collagen to form a triple helix structure. The element can effectively promote the self-crosslinking of recombinant type III humanized collagen to form a triple helix structure.

## Description

### CROSS-REFERENCE TO THE RELATED APPLICATIONS

The present disclosure claims priority to and the benefit of Chinese Patent Application No. 202310983181.3 filed with the Patent Office of China National Intellectual Property Administration (CNIPA) on August 4, 2023 and entitled "SELF-CROSSLINKING RECOMBINANT HUMANIZED COLLAGEN POLYMER BIOMATERIAL AND PREPARATION METHOD THEREFOR", the entire contents of which are incorporated herein by reference.

### TECHNICAL FIELD

The present disclosure belongs to the technical field of synthetic biology, and specifically relates to a self-crosslinking recombinant humanized collagen polymer biomaterial and a preparation method therefor, and more specifically to a collagen self-assembled element for promoting self-crosslinking of recombinant type III humanized collagen to form a triple helix structure and a corresponding recombinant type III humanized collagen having a triple helix structure.

### BACKGROUND TECHNOLOGY

Collagen is a type of protein widely distributed in the connective tissue of the human body, and is also the most abundant protein in the human body, accounting for 25% to 35% of total protein. The main function of collagen is to preserve the extracellular environment, maintain the normal physiological functions of tissue and organs, repair body damage, etc. Collagen is a native biological resource with the biological histocompatibility, support elasticity to cells, and degradability that other polymer materials cannot match. Therefore, collagen may be widely used in industries such as pharmaceutical and cosmetics.

The natural collagen molecule can form a special supercoiled structure that is a left-handed helix with three amino acid residues as the basic repeat, where these three amino acid residues are typically Gly-X-Pro. Gly is essential for formation of hydrogen bonds in collagen and does not have a side chain *per se,* thereby allowing collagen to be piled up tightly. At a higher structural level, collagen supercoils will further associate to form collagen fibrils. In organisms, the synthesis and modification of collagen begins with procollagen, involves many chemical changes such as hydroxylation, glycosylation, and crosslinking, and is subject to intricate regulations by a variety of biological enzymes. In addition to the collagen chain, procollagen further contains spherical heads and tails. Without these heads and tails, the collagen chain would not fold into a correct triple helix and would thus lack the biological activity of collagen. Therefore, collagen prepared according to the original gene sequence cannot be spontaneously organized to form a correct spatial structure *in vitro.* Such difficulties seriously hinder the development and production of humanized collagen.

People have conducted appropriate exploration. For example, Cited Literature 1 discloses that a sequence such as the hinge-region amino acids GPPGPCCGGG (SEQ ID NO.15) may be added at the C-terminus which expresses a polypeptide to aid gel formation. However, Cited Literature 2 discloses that if the above hinge-region amino acids set forth in SEQ ID NO.15 are added at the C-terminus of the polypeptide sequence provided therein, this would cause the polypeptide to form a gelatinous precipitate during fermentation culture, resulting in a failure to be dissolved and purified, so the yield is significantly reduced.

Therefore, there is currently an urgent need for a method for promoting crosslinking of recombinant type III humanized collagen to form a triple helix structure, such that the recombinant type III humanized collagen could be mass-produced by a biosynthetic method, have a triple helix structure, and exert its particular functions.

In addition, circular dichroism spectroscopy is a technique used to detect the secondary structure of proteins. When it is used to detect natural collagen under appropriate detection conditions, an absorption peak near 221 nm will appear. This absorption peak is somewhat related to its triple helix structure, but it is also subject to the content of proline and hydroxyproline, etc. (the polyproline itself shows a strong peak at 221 nm, but it does not form a triple helix). As such, the circular dichroism spectroscopy cannot serve as direct evidence of the triple helix structure. However, due to the simplicity of the experimental detection by circular dichroism spectroscopy, it can be used to assist in characterizing the higher-order structure of collagen. For different collagens, the experimental conditions and results by circular dichroism spectroscopy vary significantly. This is primarily because the sequence length and amino acid composition (especially the content of proline and hydroxyproline) of recombinant collagen are different from those of full-length native collagen, and thus the detection conditions (temperature, concentration, solution formula, etc.) for circular dichroism spectroscopy are inconsistent from one collagen to another. In order to characterize whether collagen can form a triple helix (for example, collagen denatured in production or with design defects cannot form a triple helix structure at all), the collagen may be characterized by the circular dichroism spectroscopy under different conditions.

### Citation List:

Cited Literature 1: Yao J, Yanagisawa S, Asakura T. Design, expression and characterization of collagen-like proteins based on the cell adhesive and crosslinking sequences derived from native collagens. J Biochem. 2004 Nov; 136(5):643-9.
Cited Literature 2: CN 109593126 B.

### CONTENT OF THE INVENTION

### Problem to be solved by the invention

At present, existing technologies have conducted appropriate but still inadequate studies on, for example, the problem that collagen prepared according to the original gene sequence can hardly organize spontaneously and form a correct spatial structure *in vitro,* resulting in its inability to effectively exert physiological functions. In view of this, the present disclosure provides a collagen self-assembled element capable of promoting self-crosslinking of recombinant type III humanized collagen to form a triple helix structure, specifically, incorporating a hinge-region amino acid sequence of human type III collagen at the terminus of the amino acid sequence of the recombinant type III humanized collagen to help the recombinant type III humanized collagen to self-crosslink and form a triple helix structure, and the recombinant type III humanized collagen biomaterial having the triple helix structure could be smoothly prepared by a biosynthetic method.

### Solution used to solve the problem

According to the first aspect of the present disclosure, there is provided a collagen self-assembled element, wherein the collagen self-assembled element includes a sequence set forth in any one of the following (i) to (iii):
(i) at least 21 consecutive amino acids at C-terminus of an amino acid sequence set forth in SEQ ID NO.1;
(ii) an amino acid sequence obtained by substituting, deleting or adding one or more amino acids in the amino acid sequence set forth in (i) and retaining a function of promoting self-crosslinking of a recombinant type III humanized collagen to form a triple helix structure; and
(iii) an amino acid sequence having at least 90%, 92%, 95%, 96%, 97%, 98% or 99% identity to the amino acid sequence set forth in (i) and retaining a function of promoting self-crosslinking of a recombinant type III humanized collagen to form a triple helix structure.

In some embodiments, the sequence set forth in (i) is an amino acid sequence set forth in SEQ ID NO.1, SEQ ID NO.2, SEQ ID NO.3 or SEQ ID NO.4.

According to the second aspect of the present disclosure, there is provided use of the collagen self-assembled element according to the first aspect of the present disclosure in promotion of self-crosslinking of a recombinant type III humanized collagen to form a triple helix structure.

In some embodiments, the collagen self-assembled element functions by being present at a terminus of an amino acid sequence of the recombinant type III humanized collagen;
preferably, the terminus of the amino acid sequence of the recombinant type III humanized collagen is C-terminus.

According to the third aspect of the present disclosure, there is provided a recombinant type III humanized collagen, wherein the recombinant type III humanized collagen includes the collagen self-assembled element according to claim 1 or 2 at a terminus thereof;
preferably, the terminus of the recombinant type III humanized collagen is C-terminus.

In some embodiments, the recombinant type III humanized collagen includes a sequence set forth in any one of the following (a) to (c):
(a) an amino acid sequence set forth in SEQ ID NO.6, SEQ ID NO.7, SEQ ID NO.8 or SEQ ID NO.9;
(b) an amino acid sequence obtained by substituting, deleting or adding one or more amino acids in the amino acid sequence set forth in (i) and retaining an ability to self-crosslink and form a triple helix structure; and
(c) an amino acid sequence having at least 90%, 92%, 95%, 96%, 97%, 98% or 99% identity to the amino acid sequence set forth in (i) and retaining an ability to self-crosslink and form a triple helix structure.

According to the fourth aspect of the present disclosure, there is provided a polynucleotide, wherein the polynucleotide encodes the collagen self-assembled element according to the first aspect of the present disclosure, or encodes the recombinant type III humanized collagen according to the third aspect of the present disclosure.

In some embodiments, the polynucleotide includes a sequence set forth in the following (z₁) or (z₂):
(z₁) a nucleotide sequence set forth in SEQ ID NO.11, SEQ ID NO.12, SEQ ID NO.13 or SEQ ID NO.14;
(z₂) a nucleotide sequence that hybridizes with the nucleotide sequence set forth in (z₁) under stringency conditions and encodes a protein having an ability to self-crosslink and form a triple helix structure, the stringency conditions being medium stringency conditions, medium-high stringency conditions, high stringency conditions or very high stringency conditions.

According to the fifth aspect of the present disclosure, there is provided a recombinant expression vector, wherein the recombinant expression vector includes the polynucleotide according to the fourth aspect of the present disclosure.

According to the sixth aspect of the present disclosure, there is provided a recombinant host cell, wherein the recombinant host cell includes the recombinant expression vector according to the fifth aspect of the present disclosure.

According to the seventh aspect of the present disclosure, there is provided a preparation method for the recombinant type III humanized collagen according to the third aspect of the present disclosure, wherein the preparation method includes the following steps:
S1: constructing a recombinant expression vector including the polynucleotide according to the fourth aspect that encodes the recombinant type III humanized collagen according to the third aspect, and constructing a recombinant host cell by transformation;
S2: culturing the recombinant host cell obtained in S1 in a medium and producing a protein;
S3: harvesting and purifying the protein, preferably purifying the protein by a Ni column and/or anion exchange chromatography; and
S4: optionally subjecting the protein to enzyme digestion, preferably subjecting the protein to enzyme digestion with a tobacco etch virus (TEV) protease.

### Effects of the invention

By implementing the above-mentioned technical solutions, the present disclosure achieves the following technical effects:
First of all, the present disclosure provides a collagen self-assembled element. By incorporating this collagen self-assembled element at the terminus of the amino acid sequence of the recombinant type III humanized collagen, namely, incorporating a hinge-region amino acid sequence of human type III collagen, it is possible to effectively help the recombinant type III humanized collagen to self-crosslink and form a triple helix structure, thereby significantly improving the availability of the recombinant type III humanized collagen prepared by a biosynthetic method.

Furthermore, the recombinant type III humanized collagen including a collagen self-assembled element (namely, including a hinge-region amino acid sequence of human type III collagen) at the terminus thereof as provided herein can form a good triple helix structure, which is supported by the detection results of the circular dichroism spectroscopy. Moreover, the recombinant type III humanized collagen could be mass-produced by a biosynthetic method.

### DESCRIPTION OF THE DRAWINGS

FIG. 1 shows the electrophoresis test results during the preparation of the recombinant type III humanized collagen TE16c.
FIG. 2 shows the electrophoresis test results during the preparation of the recombinant type III humanized collagen C3T16H1.
FIG. 3 shows the electrophoresis test results during the preparation of the recombinant type III humanized collagen C3T16H2.
FIG. 4 shows the electrophoresis test results during the preparation of the recombinant type III humanized collagen C3T16H3.
FIG. 5 shows the electrophoresis test results during the preparation of the recombinant type III humanized collagen C3T16H4.
FIG. 6 shows the results of circular dichroism spectroscopy and UV scanning analysis of the recombinant type III humanized collagens C3T16H1, C3T16H2, C3T16H3, C3T16H4 and TE16c, with positive peaks detected near 221 nm.

### SPECIFIC IMPLEMENTATIONS

The embodiments of the present disclosure will be described below, but the present disclosure is not limited thereto. The present disclosure is not limited to the elements described below. Various modifications may be made within the scope as claimed in the invention. Moreover, the embodiments or examples obtained by appropriately combining the technical means disclosed respectively in different embodiments or examples are also encompassed in the technical scope of the present disclosure.

The numerical range represented by "numerical value A to numerical value B" or "numerical value A - numerical value B" used in the present disclosure refers to the range including the endpoint values A and B.

In the present disclosure, the numerical range represented by "or more" or "or less" refers to a numerical range including the number itself.

In the present disclosure, the term "optional" or "optionally" means that the event or case described subsequently may or may not occur, and the description includes the case where the event occurs and the case where the event does not occur.

In the present disclosure, the term "a" or "an" or "the" may refer to "one," or refer to "one or more," "at least one," and "one or more than one."

In the present disclosure, the term "including," "having," "comprising," or "containing" is intended to be inclusive or open-ended, and does not exclude additional or unrecited elements or methods and steps. At the same time, the term "including," "having," "comprising," or "containing" may also be intended to be close-ended and exclude additional or unrecited elements or methods and steps.

In the present disclosure, the term "about" may indicate that a value includes the standard deviation of an error caused by the device or method used to measure the value. The numerical ranges and parameters used to define the present disclosure are all approximate numerical values, and the numerical values involved in the specific examples have been presented herein as accurately as possible. However, any numerical value inevitably contains a standard deviation caused by the aforementioned testing device or method in nature. Therefore, it should be understood that all the ranges, quantities, numerical values, and percentages used herein have already been modified by "about," unless otherwise expressly stated. The term "about" used herein generally means that the actual value is within ±10%, ±5%, ±3%, ±1%, or ±0.5% of a particular numerical value or range.

In the present disclosure, the terms "polypeptide" and "protein" may interchangeably refer to a string of at least two amino acid residues linked to each other via a covalent bond (such as a peptide bond), and may be a recombinant polypeptide, a native polypeptide or a synthetic polypeptide. The polypeptide may be linear or branched. It may include modified amino acids, and may be interrupted by non-amino acids. The term also includes amino acid polymers that have been modified (e.g., disulfide bond formation, glycosylation, lipidation, acetylation, phosphorylation, or any other manipulations, such as conjugation with a labeling component).

In the present disclosure, the term "amino acid" may include natural amino acids, unnatural amino acids, amino acid analogs, and all D and L stereoisomers thereof.

In the present disclosure, amino acid modifications may include modifications to native sequences, such as modifications to functional groups, intramolecular covalent bonding (e.g., ring formation between side chains), methylation, acylation, ubiquitination, phosphorylation, aminohexylation, and biotinylation.

In the present disclosure, amino acid deletion may refer to the deletion of 1, 2, or 3 or more amino acids from an amino acid sequence, as long as the altered sequence fully or partially retains the activity of the original amino acid sequence.

In the present disclosure, amino acid addition may refer to the addition of 1, 2, or 3 or more amino acids at the C-terminus, N-terminus, or any position between the C-terminus and N-terminus of an amino acid sequence, as long as the altered sequence fully or partially retains the activity of the original amino acid sequence.

In the present disclosure, amino acid substitution may refer to the substitution of an amino acid at a certain position of an amino acid sequence with an additional amino acid, as long as the altered sequence fully or partially retains the activity of the original amino acid sequence. Amino acid substitutions may be conservative amino acid substitutions, which mean that several amino acids are substituted with amino acids of similar or approximate properties to form peptides (conservative variant peptides) as compared to the original amino acid sequence. For example, these conservative variant peptides may be produced by the following substitutions of amino acids: a substitution of Ala with Val, Leu or Ile, a substitution of Arg with Lys, Gln, Asn or His, a substitution of Asn with Gln, His, Lys or Arg, a substitution of Asp with Glu or Asn, a substitution of Cys with Ser or Ala, a substitution of Gln with Asn or Glu, a substitution of Glu with Asp or Gln, a substitution of Gly with Ala, a substitution of His with Asn, Lys, Gln or Arg, a substitution of Ile with Leu, Met, Ala, Val, Phe or norleucine (Nle), a substitution of Leu with Ile, Met, Ala, Val, Phe or Nle, a substitution of Lys with Asn, Gln or Arg, a substitution of Met with Ile, Leu or Phe, a substitution of Phe with Leu, Val, Ile, Ala or Tyr, a substitution of Pro with Ala, a substitution of Ser with Thr, a substitution of Thr with Ser or Val, a substitution of Trp with Phe or Tyr, a substitution of Tyr with Trp, Phe, Thr or Ser, and a substitution of Val with Phe, Ala, Met, Ile, Leu or Nle. Amino acid substitutions may also be non-conservative amino acid substitutions.

In the present disclosure, the terms "sequence identity" and "percent identity" refer to the percentage of nucleotides or amino acids that are the same (i.e., identical) between two or more polynucleotides or polypeptides. The sequence identity between two or more polynucleotides or polypeptides may be determined by aligning the nucleotide sequences of polynucleotides or the amino acid sequences of polypeptides and scoring the number of positions at which nucleotide or amino acid residues are identical in the aligned polynucleotides or polypeptides, and comparing the number of these positions with the number of positions at which nucleotide or amino acid residues are different in the aligned polynucleotides or polypeptides. Polynucleotides may differ at one position by, e.g., containing a different nucleotide (i.e., substitution or mutation) or deleting a nucleotide (i.e., insertion or deletion of a nucleotide in one or two polynucleotides). Polypeptides may differ at one position by, e.g., containing a different amino acid (i.e., substitution or mutation) or deleting an amino acid (i.e., insertion or deletion of an amino acid in one or two polypeptides). The sequence identity may be calculated by dividing the number of positions at which nucleotide or amino acid residues are identical by the total number of amino acid residues in the polynucleotides or polypeptides. For example, the percent identity may be calculated by dividing the number of positions at which nucleotide or amino acid residues are identical by the total number of nucleotide or amino acid residues in the polynucleotides or polypeptides, and multiplying the result by 100.

In the present disclosure, the term "medium stringency conditions," "medium-high stringency conditions," "high stringency conditions" or "very high stringency conditions" describes the conditions for hybridization and washing of nucleic acids. Guidance for hybridization reactions is described in Current Protocols in Molecular Biology, John Wiley & Sons, N.Y. (1989), 6.3.1-6.3.6, which is incorporated herein by reference. This literature describes both a method involving water and a method involving no water and either of the methods may be used. For example, the specific hybridization conditions are as follows: (1) the low stringency hybridization conditions refer to 6× sodium chloride/sodium citrate (SSC) at about 45°C, and then washing twice in 0.2× SSC and 0.1% sodium dodecyl sulfate (SDS) at 50°C or higher (for the low stringency conditions, the washing temperature can be raised to 55°C); (2) the medium stringency hybridization conditions refer to 6× SSC at about 45°C, then washing one or more times in 0.2× SSC and 0.1% SDS at 60°C; (3) the high stringency hybridization conditions refer to 6× SSC at about 45°C, and then washing one or more times in 0.2× SSC and 0.1% SDS at 65°C, which are preferred; and (4) the very high stringency hybridization conditions refer to 0.5 M sodium phosphate and 7% SDS at 65°C, and then washing one or more times in 0.2× SSC and 1% SDS at 65°C.

In the present disclosure, the term "polynucleotide" refers to a polymeric form of nucleotides of any length, regardless of deoxyribonucleotides or ribonucleotides or analogs thereof. A polynucleotide may have any three-dimensional structure and may perform any known or unknown function.

In the present disclosure, the term "recombinant expression vector" refers to a DNA structure for expressing, *e.g.,* a polynucleotide encoding the desired polypeptide. The recombinant expression vector may include, for example, containing i) a set of genetic elements having a regulatory effect on gene expression, such as promoters and enhancers; ii) a structure or coding sequence transcribed into mRNA and translated into a protein; and iii) an appropriate transcription subunits of transcriptional and translational initiation and termination sequences. The recombinant expression vector is constructed in any suitable manner. The nature of the vector is not important, and any vector may be used, including plasmids, viruses, phages, and transposons. Possible vectors for use in the present disclosure include, but are not limited to, chromosomes, non-chromosomes, and synthetic DNA sequences, for example, viral plasmids, bacterial plasmids, phage DNAs, yeast plasmids, and vectors derived from combinations of plasmids and phage DNAs, and DNAs from viruses such as lentivirus, retroviruses, cowpox, adenoviruses, avian pox, baculoviruses, simian virus 40 (SV40), and pseudorabies virus.

In the present disclosure, the term "recombinant host cell" refers to a cell into which an expression vector has been introduced. Host cells may include bacterial, microbial, plant or animal cells. Easily transformed bacteria include members of the Enterobacteriaceae family, such as strains of *E. coli* or *Salmonella*; *Bacillaceae* family such as *Bacillus subtilis*; Pneumococcus; Streptococcus and *Haemophilus influenzae.* Suitable microorganisms include *Saccharomyces cerevisiae* and *Pichia pastoris.* Suitable animal host cell lines include CHO (Chinese Hamster Ovary cell line) and non-secreting myeloma (NS0) cells.

In the present disclosure, the term "codon optimization" means that the nucleotide sequence encoding the polypeptide has been configured to contain codons preferred by the host cell or organism to improve the gene expression in the host cell or organism and increase translation efficiency.

Unless otherwise defined, the other technical and scientific terms used herein have the same meanings as typically understood by one of ordinary skill in the art of the present disclosure.

The technical solutions provided herein will be described in more details below:

### Collagen self-assembled element

The present disclosure provides a collagen self-assembled element that is effective in promoting self-crosslinking of recombinant type III humanized collagen to form a triple helix structure.

In some embodiments, the collagen self-assembled element includes a sequence set forth in any one of the following (i) to (iii):
(i) at least 21 consecutive amino acids at C-terminus of an amino acid sequence set forth in SEQ ID NO.1;
(ii) an amino acid sequence obtained by substituting, deleting or adding one or more amino acids in the amino acid sequence set forth in (i) and retaining a function of promoting self-crosslinking of a recombinant type III humanized collagen to form a triple helix structure; and
(iii) an amino acid sequence having at least 90%, 92%, 95%, 96%, 97%, 98% or 99% identity to the amino acid sequence set forth in (i) and retaining a function of promoting self-crosslinking of a recombinant type III humanized collagen to form a triple helix structure.

SEQ ID NO.1: GERGSEGSPGHPGQPGPPGPPGAPGPCCGG, which is a peptide fragment of the human type III collagen.

It has been unexpectedly discovered in the present disclosure that collagen self-assembled elements of different lengths have different abilities to promote self-crosslinking of recombinant type III humanized collagen to form a triple helix structure.

In some specific embodiments, the amino acid sequence of the collagen self-assembled element includes the sequence set forth in SEQ ID NO.1, SEQ ID NO.2, SEQ ID NO.3 or SEQ ID NO.4.

In some more specific embodiments, the amino acid sequence of the collagen self-assembled element is the sequence set forth in SEQ ID NO.1, SEQ ID NO.2, SEQ ID NO.3 or SEQ ID NO.4.
SEQ ID NO.2: GSEGSPGHPGQPGPPGPPGAPGPCCGG.
SEQ ID NO.3: GSPGHPGQPGPPGPPGAPGPCCGG.
SEQ ID NO.4: GHPGQPGPPGPPGAPGPCCGG.

In order that the collagen self-assembled element functions effectively in promoting self-crosslinking of the recombinant type III humanized collagen to form a triple helix structure, in some embodiments, the collagen self-assembled element is located at the terminus, preferably the C-terminus, of the recombinant type III humanized collagen.

The recombinant type III humanized collagen according to the present disclosure refers to a full-length or partial amino acid sequence fragment encoded by a human type III collagen gene prepared by the DNA recombinant technique, or a combination containing functional fragments of the human type III collagen.

In some specific embodiments, the recombinant type III humanized collagen contains a collagen self-assembled element at a terminus of the amino acid sequence thereof, which can be realized by designing an amino acid sequence of the recombinant type III humanized collagen containing a collagen self-assembled element at the terminus thereof, and a gene sequence thereof; constructing an expression vector containing the gene sequence, and transforming the expression vector into a host cell; subsequently, subjecting the host cell to fermentation culture, and inducing expression of the recombinant type III humanized collagen.

### Recombinant Type III Humanized Collagen

The present disclosure provides a recombinant type III humanized collagen, including a collagen self-assembled element at a terminus thereof. The collagen self-assembled element is as described above. Because of containing a collagen self-assembled element, the recombinant type III humanized collagen provided herein can self-crosslink and form a triple helix structure after expression, and this protein can be mass-produced by a biosynthetic method.

To be more conducive to formation of a triple helix structure, the collagen self-assembled element is located at the C-terminus of the recombinant type III humanized collagen.

In some specific embodiments, the recombinant type III humanized collagen includes a sequence set forth in any one of the following (a) to (c):
(a) an amino acid sequence set forth in SEQ ID NO.6, SEQ ID NO.7, SEQ ID NO.8 or SEQ ID NO.9;
(b) an amino acid sequence obtained by substituting, deleting or adding one or more amino acids in the amino acid sequence set forth in (i) and retaining an ability to self-crosslink and form a triple helix structure; and
(c) an amino acid sequence having at least 90%, 92%, 95%, 96%, 97%, 98% or 99% identity to the amino acid sequence set forth in (i) and retaining an ability to self-crosslink and form a triple helix structure.

In some preferred embodiments, the amino acid sequence of the recombinant type III humanized collagen is the sequence set forth in SEQ ID NO.6, SEQ ID NO.7, SEQ ID NO.8 or SEQ ID NO.9.

### Polynucleotide, Recombinant Expression Vector, Recombinant Host Cell

The present disclosure provides a polynucleotide encoding the collagen self-assembled element and the recombinant type III humanized collagen as described above.

In some embodiments, the polynucleotide encoding the recombinant type III humanized collagen includes a sequence set forth in the following (z₁) or (z₂):
(z₁) a nucleotide sequence set forth in SEQ ID NO.11, SEQ ID NO.12, SEQ ID NO.13 or SEQ ID NO.14;
(z₂) a nucleotide sequence that hybridizes with the nucleotide sequence set forth in (z₁) under stringency conditions and encodes a protein having an ability to self-crosslink and form a triple helix structure, the stringency conditions being medium stringency conditions, medium-high stringency conditions, high stringency conditions or very high stringency conditions.

The present disclosure provides a recombinant expression vector including the aforesaid polynucleotide.

The expression vector used herein is stable and spontaneously replicable in various prokaryotic or eukaryotic host cells, such as conventional plasmids (pET series), shuttle vector PNV 18.1, phages or virus vectors in this field. The sequence of the polynucleotide described above in the present disclosure is cloned into the vector through molecular biological operations such as enzyme digestion and ligation on the vector to construct a recombinant expression vector.

The present disclosure provides a recombinant host cell including the aforesaid recombinant expression vector.

The host cell according to the present disclosure is selected from prokaryotic cells, yeast and eukaryotic cells, and may further be selected from *E. coli, Rhodococcus ruber, Bacillus subtilis* and yeast. The recombinant expression vector described above in the present disclosure is transformed into a host cell to obtain corresponding genetically engineered bacteria.

### Preparation Method for Recombinant Type III Humanized Collagen

The present disclosure provides a preparation method for the recombinant type III humanized collagen as described above, wherein the preparation method includes the following steps:
S1: constructing a recombinant expression vector including a polynucleotide encoding the recombinant type III humanized collagen, and constructing a recombinant host cell by transformation;
S2: culturing the recombinant host cell in a medium and producing a protein;
S3: harvesting and purifying the protein, preferably purifying the protein by a Ni column and/or anion exchange chromatography; and
S4: optionally subjecting the protein to enzyme digestion, preferably subjecting the protein to enzyme digestion with a tool enzyme for collagen.

### Examples

The embodiments of the present disclosure will be described in detail below with reference to the examples. However, a person skilled in the art shall appreciate that the following examples are merely intended to illustrate the present disclosure and shall not be regarded as limiting the scope of the present disclosure. Where no specific conditions are indicated in the examples, conventional conditions or those recommended by manufacturers are followed. All the materials or instruments are commercially-available conventional products, unless otherwise specified.

### Example 1: Construction and Expression of Recombinant Type III Humanized Collagens

1. The amino acid sequences of different lengths in the hinge region of the human type III collagen were combined with the amino acid sequence of the recombinant type III humanized collagen (TE16c described in the present inventors' earlier patent ZL201811438582.6) to obtain recombinant type III humanized collagens containing hinge regions of different lengths.
   (1) Amino Acid Sequence
      Amino acid sequence of recombinant type III humanized collagen TE16c: 
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
 
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
(SEQ ID NO.5).
      Amino acid sequence of recombinant type III humanized collagen C3T16H1: 
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGSEGSPGHPGQPGPPGPPGAPGPCCGG (SEQ ID NO.6).
      Amino acid sequence of recombinant type III humanized collagen C3T16H2: 
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GSEGSPGHPGQPGPPGPPGAPGPCCGG (SEQ ID NO.7).
      Amino acid sequence of recombinant type III humanized collagen C3T16H3: 
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
 
GERGAPGFRGPAGPNGIPGEKGPAGERGAPGERGAPGFRGPAGPNGIPGEKGPAGERGAP
GSPGHPGQPGPPGPPGAPGPCCGG (SEQ ID NO.8).
      Amino acid sequence of recombinant type III humanized collagen C3T16H4: 
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GERGAPGFRGPAGPNGIPGEKGPAGERGAPGERGAPGFRGPAGPNGIPGEKGPAGERGAP
GHPGQPGPPGPPGAPGPCCGG (SEQ ID NO.9).
      In the above sequences, the underlined portions were amino acid sequences of different lengths in the hinge regions of the human type III collagen, namely, collagen self-assembled elements of different lengths.
   (2) Nucleotide Sequence
      Nucleotide sequence of recombinant type III humanized collagen TE16c: 
ggtgaacgtggtgcaccaggttttcgtggtccggcaggtccgaatggaattccgggtgagaaaggaccggctggtgagcgtggtgcgcc
gggtgaacgtggagcgcctggttttcgtggcccagcaggtccgaacggtattcctggtgaaaaaggtccggcgggagagcgtggtgcac
cgggtgaacgcggtgcaccgggatttcgtggtccagcaggaccgaatggtatccctggtgaaaaaggaccggcaggtgagcgtggagc
gccaggtgaacgtggcgcaccgggttttcgtggaccggcaggcccgaatggtattccgggtgaaaaaggcccggcaggtgaacgtggt
gccccgggtgaacgtggtgcgcctggatttcgtggcccggcaggaccgaacggtatccctggagaaaaaggtcctgcaggtgagcgcg
gtgcgccgggcgagcgtggtgcccctggttttcgcggtccggcaggccctaatggtattcctggagaaaaaggccctgcaggtgaacgc
ggagcaccgggtgagcgtggcgcacctggttttcgtggtcctgcaggcccgaacggtattccgggcgaaaaaggtccagcaggtgaacg
tggtgctccgggtgaacgtggtgcacctggatttcgcggtcctgctggtccgaatggtattccaggtgaaaaaggtccggcaggagagcgt
ggagcaccgggagaacgtggtgcaccgggctttcgtggtccggccggtcctaacggtatcccaggtgaaaaaggtccggccggcgagc
gtggcgcccctggtgagcgtggtgctcctggttttcgtggtccggctggtccgaacggaattcctggtgagaaaggtccggctggcgaacg
tggtgcaccgggtgaacgtggtgcaccgggtttccgtggtccggcgggtcctaatggtatcccgggtgaaaaaggtccggcaggtgaac
gtggtgcaccgggtgaacgtggtgcaccgggttttcgcggaccggcaggacctaatggtattccgggagaaaaaggacctgcgggtgaa
cgtggtgcaccgggtgaacgtggtgcaccgggttttcgtggtccggcaggtcctaatggaattcctggagagaaaggacctgcaggtgaa
cgtggtgcaccgggtgaacgtggtgcaccgggttttcgtggtccggcaggtccaaatggtattccgggtgaaaaaggtccggcaggtgaa
cgtggtgcaccgggtgaacgtggtgcaccgggttttcgtggtccggcaggtccgaatggcattcctggtgaaaaaggtccggcaggtgaa
cgtggtgcaccgggtgaacgtggtgcaccgggttttcgtggtccggcaggtccgaatggtattccgggtgaaaaaggtccggcaggtgaa
cgtggtgcaccg (SEQ ID NO.10).
      Nucleotide sequence of recombinant type III humanized collagen C3T16H1: 
ggagaaagggggcgccaggtttccgtggtccggccggcccgaacggcatcccgggtgaaaaggaccggcgggtgaacgtggtgc
gccgggagaacgtggagcgccgggctttcgtggtccggcgggtcccaacggcattccgggtgagaagggcccggccggtgaacgtgg
tgctccgggcgagcgtggtgccccgggcttccgtggccccgccggtccgaacggcatcccgggtgagaaaggtccggctggcgagcgt
ggcgcacctggcgagcgcggcgcgccaggttttcgtggtccggcgggtcctaatggtattccgggcgaaaagggtccggcaggcgaac
gtggtgctccgggtgagcgtggtgctccgggttttcgtggtccggcgggtcccaacggcatcccgggcgagaaaggtccggcgggtgag
cgcggtgcaccaggtgagcgcggcgcgcctggttttagaggtccggcgggtccgaacggtattccgggtgagaagggtccggcaggcg
aacgcggagcgccgggcgaacgtggggccccgggattccgtggaccggcaggtccgaacggcattccgggcgaaaaaggcccggc
gggtgagcgcggcgcgcctggcgaacgtggcgctccgggctttcgtggcccggcgggcccgaatggtatcccgggtgaaaaaggccc
agccggtgaacgcggtgcaccgggcgaacgtggtgcccccggattccgcggtcctgctggtccgaacggcattccgggcgaaaagggt
ccggctggtgaacgaggcgcaccgggcgagcgcggggctccgggcttccgtggaccagcgggcccgaatggtattcctggcgaaaag
ggtccggcaggtgagcggggcgcaccgggtgaacgtggcgcacccggcttccgcggaccggcgggcccgaatggtatcccgggtga
aaaaggtccggcgggtgagcgtggtgcgccgggtgagcgtggcgcaccaggttttcgcggtccggcaggcccgaatggtatcccgggt
gagaagggcccagcgggtgagcgtggtgcgccgggtgaacgcggggcccctggcttccgtggtccggcgggtccgaacggtatcccg
ggtgaaaaaggcccagcaggcgagcgcggtgctccgggtgagcgtggagctccgggctttcgtggcccggccggtccgaacggcattc
cgggtgaaaagggtccggcgggtgagcgtggtgcgccaggtgagcgtggtgctccgggtttccgtggtccggcgggtccgaacggcat
cccgggtgagaaaggtccggcaggcgaacgtggcgcgccaggcgaacgtggggcgccaggtttccgcggtccagcaggtccgaatg
gcatcccgggtgagaaaggcccggcgggtgagcgcggtgcaccgggtgagcgcggtagcgaaggttccccgggtcacccgggtcag
ccgggcccgccgggtccgcccggcgccccgggtccgtgttgcggtggt (SEQ ID NO.11).
      Nucleotide sequence of recombinant type III humanized collagen C3T16H2: 
ggagaaagggggcgcctggctttcgtggtccggctggtccgaacggtattccgggtgaaaaggtccggcgggcgagcgcggtgcac
cgggtgagcgtggtgcccctggtttccgtggcccagctggcccgaacggcatcccgggtgagaaaggcccagcgggcgagcgtggtg
cgccgggtgagcgtggagcgccaggattccgtggcccggctggcccgaacggtattccgggtgaaaagggcccggcgggcgaaagg
ggcgcgccaggagaaagaggtgcgcccggcttccgcggtccggctggcccgaacggcatcccgggtgaaaaggtccggcaggtga
aagaggcgcgccgggcgagcgtggtgcaccgggctttcgtggtccggctggcccgaacggcatcccgggcgagaaaggcccggcgg
gtgaacgtggcgcgccaggtgaacgtggtgcgcctggcttccgtggtccggcgggtccgaatggcatcccgggggagaaaggtccggc
aggtgagcgcggtgcaccgggtgagcgtggtgcaccgggattccgcggccctgcgggcccgaacggcatcccgggtgaaaaaggtcc
ggcaggtgaacgcggtgccccaggcgaacgtggcgcgcctggctttcgtggtcctgccggtcctaatggtattccgggcgaaaagggcc
cggcgggcgaacgtggcgccccaggcgaacgtggtgcgccgggttttcgcggaccggcgggtccgaatggtatcccgggcgaaaagg
gtccggccggtgagcgtggcgcaccgggggaacgtggcgcgccaggcttccgcggcccggcaggcccgaacggcatcccgggcga
gaagggtccggcgggtgagcgtggcgctccgggtgagcgcggtgccccgggatttcgcggtccggcgggtccgaatggcatcccggg
cgagaagggtccggcgggcgagcgcggtgctccgggtgaacgtggtgccccgggcttccgtggtcctgccggtccgaacggtattcctg
gtgagaaaggcccagccggtgaacgcggtgcaccgggtgaaagaggtgcgccagggtttcgtggcccggcgggtcctaatggtattcc
gggcgaaaaaggaccggcgggtgagcgtggtgcgccgggcgagcgcggtgcaccgggcttccgtggcccggcgggtccgaatggc
 
attccgggtgaaaaggggccggctggcgagcgtggtgctccgggtgaacgtggcgcaccggggtttcgcggtcccgcgggccccaac
ggcatcccgggtgagaagggcccggcgggtgagcgcggcgcgccaggcgaacgtggagcgccgggattccgcggcccggccggc
cccaacggtattccgggtgagaagggtccggctggagagcggggcgcgccgggtagcgaaggttccccgggccacccgggtcagcc
gggccctccaggtccgcctggtgcgcctggcccttgttgcggtggt (SEQ ID NO.12).
      Nucleotide sequence of recombinant type III humanized collagen C3T16H3: 
ggagaaagggggcgccagggtttcgcggtccggcgggtccgaacggtattccgggcgagaagggtccggcgggtgagcgtggagc
gccgggtgagaggggcgcgccgggcttccgtggcccagcgggaccgaacggtattccaggtgaaaaaggtccggctggcgagcgtg
gtgcgccgggtgaacgtggggcaccgggctttcgtggcccggcgggccccaacggtattccgggtgaaaagggcccggccggtgagc
gtggtgcgcctggtgagcgcggagcgccgggattccgtggcccagcgggaccgaatggcatcccgggtgaaaaaggtccggcgggc
gagcgtggcgctccgggcgaacgtggcgcaccgggttttcgtggccctgcgggtccgaatggcatcccgggtgaaaagggcccagcag
gtgaacgtggtgctccgggtgagcgcggtgcgccgggcttccgcggtccggcgggtcctaatggcatcccgggtgagaagggtccagc
cggtgaacgtggcgcaccgggcgagcggggtgccccgggatttcgtggtccggctggcccaaatggtattccgggtgaaaaaggtccg
gctggcgaacgtggcgcaccgggtgagcgtggtgctccgggattccgcggtccggcgggaccgaatggtatcccgggtgagaaaggtc
cggccggtgaacgtggcgccccgggcgagagaggtgcccctggcttccgcggtccggcaggtccgaacggtatcccgggtgagaaag
gtccggctggtgagcgtggcgcgccaggtgagcgtggtgcgcctggtttccgcggcccggcaggtcctaacggtattcctggcgaaaag
ggtcctgccggtgagcgtggagcccctggcgaacgtggtgcgccaggtttccgtggtccggctggccccaacggcatcccgggcgaga
aaggtccggcgggtgagcgtggcgctccgggggagcgcggcgccccgggattcaggggtccggcgggtccgaacggcatcccgggt
gagaagggcccggccggcgagcgcggtgccccaggcgagcgcggcgctccgggttttcgtggtccggcaggccccaacggcatccc
gggcgaaaaaggtccggctggcgaacgcggtgctccgggtgaacgtggtgcaccgggcttccgtggcccagcggggccgaatggcat
tccgggcgaaaagggtccggcgggtgagcgcggtgcaccgggggagcgtggtgcacctggctttcgtggtccggcgggtcccaacgg
catcccgggcgagaaaggtccggcgggtgaacgcggtgccccgggcgaacgtggcgcgccaggttttcgtggcccggcgggtccgaa
cggcattccgggcgaaaagggccctgcgggtgaacggggcgcaccgggcagcccgggccacccgggtcagccgggcccaccgggt
ccgcctggggctccgggtccgtgttgcggtggt (SEQ ID NO.13).
      Nucleotide sequence of recombinant type III humanized collagen C3T16H4: 
ggagaaagggggcgccgggattccgtggtcctgctggtccgaacggcatccccggtgaaaagggcccggcgggggagcgtggagc
cccgggagaacgtggtgcgccaggcttccgtggcccagcgggtccgaacggtattccgggggagaaaggcccggcgggcgaacgcg
gtgcaccaggcgaaaggggcgcgccaggattcagaggcccggcgggtccgaatggcatcccgggcgagaaaggtccggcgggtgaa
cgtggcgcgccgggtgaacgcggtgctccgggttttcgtggtccggctggcccgaacggcatcccgggcgagaaaggcccggccggt
gaacgtggagcgccaggtgagcgcggcgcgccgggcttccgtggcccagcgggcccgaacggtattccgggtgaaaagggtccggc
gggtgaacgtggcgcgccgggtgaacgtggtgcgcctggctttaggggcccagctggcccgaatggtatcccgggtgagaaaggtcca
gctggagagcgaggtgccccgggcgaacgtggtgcccctggttttcgtggtccggccggtccgaatggcatcccgggcgaaaaaggtc
cggctggggagcgtggggcaccgggcgaacggggtgcgccgggcttccgtggcccggcaggccccaacggtatcccgggtgagaag
ggtccggcgggtgagcgcggtgcaccgggcgaacgcggtgctccgggttttcgcggcccggcgggtccgaatggtattccgggcgag
aagggcccggcaggcgagcgtggtgctccaggtgaacgcggtgccccgggctttcgtggtccggcgggtcccaacggtatcccgggtg
 
agaagggtccagccggagagcgtggtgcaccaggagagcgcggtgcgcccggcttccgcggtccggcaggcccgaatggcatcccg
ggcgagaaaggtccggcaggcgagcgtggcgcaccgggagagcgtggcgcgcctggtttccgtgggccggcaggtcccaacggcatt
ccgggagaaaagggtccggcgggtgaacgtggtgcgccgggtgagcgtggcgccccgggattccgtggtccggcgggtccgaacgg
catcccgggcgagaaaggtcctgctggcgagcgtggtgcgccgggtgaacgtggtgcaccgggtttccgcggtccggcaggccccaat
ggtattccgggtgagaagggtccagcgggtgaacgtggtgcgccgggcgaacgcggtgccccgggttttcgtggtccggctggtccgaa
cggcattccgggcgaaaaaggcccggccggtgaacgcggcgcaccgggtgaacgcggtgcgccgggctttcgtggtcctgctggtcca
aacggtattccgggcgaaaagggcccggctggcgagcgtggcgcgccgggacacccgggtcagccgggtcctccgggtccgcctgg
cgcgccgggcccttgttgcggtggt (SEQ ID NO.14).
2. The gene sequences of the recombinant type III humanized collagens C3T16H1, C3T16H2, C3T16H3, and C3T16H4 were synthesized. Each of the above coding nucleotide sequences (adding an enzyme digestion site of the tool enzyme for collagen at the 5' end, where the amino acid sequence of the enzyme digestion site of the tool enzyme for collagen was ENLYFQ (SEQ ID NO.16), and its nucleotide sequence was gaaaacctgtatttccag (SEQ ID NO.17)) was inserted between the enzyme digestion sites KpnI and XhoI of the pET-28a-Trx-His expression vector. The TE16c recombinant expression plasmid was an available plasmid described in the present inventors' earlier patent ZL201811438582.6.
3. The successfully constructed expression plasmids were transformed into *E. coli* competent cells BL21 (DE3). The specific process was as follows: (1) The *E. coli* competent cells BL21 (DE3) were taken out from an ultra-low temperature refrigerator and placed on ice. When half-melted, 2 µL of plasmid to be transformed was measured and added into the *E. coli* competent cells BL21 (DE3), and mixed slightly well for 2 to 3 times. (2) The mixture was placed on ice and subjected to ice bath for 30 min, and then to heat shock in a water bath at 42°C for 45 s to 90 s. After removal, the mixture was placed on ice and subjected to ice bath for 2 min. (3) The mixture was transferred to a biological safety cabin, and 700 µL of liquid LB medium was added, and cultured for 60 min under the conditions of 37°C and 220 rpm. (4) 200 µL of bacterial solution was measured and spread uniformly onto an LB plate containing ampicillin sodium. (5) The plate was incubated in an incubator at 37°C for 15 to 17 h until colonies of uniform size grew.
4. From the transformed LB plate, 5 or 6 single colonies were picked out and put in a shake flask containing an antibiotic stock solution (the single colonies transfected with the C3T16H1, C3T16H2, C3T16H3, and C3T16H4 recombinant expression plasmids were added to an antibiotic stock solution containing kanamycin sulfate correspondingly, and the single colonies transfected with the TE16c recombinant expression plasmid were added to an antibiotic stock solution containing ampicillin sodium), and cultured in a constant-temperature shaker at 220 rpm and 37°C for 7 h. The cultured shake flask was then cooled to 16°C. After IPTG was added to induce expression for a period of time, the bacterial solution was sub-packed into centrifuge bottles, and centrifuged at 8000 rpm and 4°C for 10 min. The bacteria were collected, and the weight of the bacteria was recorded. Samples were taken for the electrophoresis test.
5. The collected bacteria were re-suspended with an equilibrium working solution (200 mM sodium chloride, 25 mM tris (hydroxymethyl)-aminomethane (Tris), 20 mM imidazole, pH8.0). The bacterial solution was cooled to ≤15°C, and subjected to homogenization with high-pressure homogenization twice (homogenized sample was taken and marked as homogenization). Upon completion, the bacterial solution was collected. The bacterial solution obtained after homogenization was sub-packed into centrifuge bottles, and centrifuged at 17000 rpm and 4°C for 30 min. The supernatant was collected. The supernatant (marked as supernatant) and precipitate (marked as precipitate) were collected for the electrophoresis test.
6. The collected supernatant was purified and enzyme digested. The specific process was as follows: (1) Capture: a. The column (Ni6FF, Cytiva) was washed at 5 column volume (CV). b. The column was equilibrated with an equilibrium solution (200 mM sodium chloride, 25 mM Tris, 20 mM imidazole, pH8.0) at 5 CV. c. Sample Loading: The collected supernatant was added to the column until the liquid was exhausted. The flow-through solution was measured for the electrophoresis test (marked as flow-through). d. Cleaning of Impurity Proteins: 25 mL of washing buffer (200 mM sodium chloride, 25 mM Tris, 20 mM imidazole, pH8.0) was added until the liquid was exhausted, and the flow-through solution resulting from impurities cleaning was measured for the electrophoresis test (marked as impurity cleaning). e. Collection of Target Proteins: 20 mL of eluent (200 mM sodium chloride, 25 mM Tris, 250 mM imidazole, pH8.0) was added, and the flow-through solution was collected (marked as elution). The protein concentration was tested by the ultraviolet-visible spectrophotometry and the protein was subjected to the electrophoresis test. f. The column was washed with 1M imidazole working solution (marked as 1M washing). g. The column was washed with purified water. (2) Enzyme digestion: The tool enzyme for collagen was added at a ratio of 20:1 between the mass of proteins and the mass of the tool enzyme for collagen, and the samples were enzyme digested at 16°C for 4 h. Samples were taken for the electrophoresis test (marked as enzyme digestion). The protein solution obtained after enzyme digestion was put into a dialysis bag, in which the dialysate was liquid A (20 mM Tris, 20 mM sodium chloride, pH8.0), dialyzed at 4°C for 2 h, then transferred to a new dialysate, and dialyzed overnight at 4°C. Samples were taken for the electrophoresis test (marked as diafiltration). (3) Polishing: a. Equilibration of Column (Capto Q, Cytiva): The column was equilibrated with liquid A (20 mM Tris, 20 mM sodium chloride, pH8.0) at a flow rate of 10 mL/min. b. Sample Loading: The flow rate was 5 ml/min. The sample was loaded and the flow-through solution was collected (marked as Q flow-through) for the electrophoresis test. c. Gradient elution of binary mobile phase system: the following gradients were set respectively: eluting with 0% (V/V) - 15% (V/V) liquid B (20 mM Tris, 1M sodium chloride, pH8.0) and 100% (V/V) - 85% (V/V) liquid A for 2 min and then maintaining at 3 CV; eluting with 15% (V/V) - 30% (V/V) liquid B and 85% (V/V) - 70% (V/V) liquid A for 2 min and then maintaining at 3 CV; eluting with 30% (V/V) - 50% (V/V) liquid B and 70% (V/V) - 50% (V/V) liquid A for 2 min and then maintaining at 3 CV; and eluting with 50% (V/V) - 100% (V/V) liquid B and 50% (V/V) - 0% (V/V) liquid A for 2 min and then maintaining at 3 CV. Samples were collected upon occurrence of characteristic peaks (marked as B elution) and subjected to the electrophoresis test. d. Cleaning of Column. e. The target protein content was tested and the protein yield was calculated. The protein was preserved at 4°C.
7. Concentration Test

### (1) Concentration test on captured protein

An appropriate amount of sample was precisely measured, diluted with eluent by 10 to 50 folds, and stirred thoroughly with a glass rod. The absorbance was determined at 280 nm using an ultraviolet-visible spectrophotometry. The protein concentration was calculated based on the following formula: C (mg/ml) = A280 × absorptivity × dilution ratio (note: the absorbance value was required to be from 0.1 to 1).

The test results of the concentration were as follows:

| Plasmid | TE16c | C3T16H1 | C3T16H2 | C3T16H3 | C3T16H4 |
|---|---|---|---|---|---|
| Absorptivity | 3.48 | 4.18 | 4.15 | 4.13 | 4.13 |
| Dilution ratio | 1 | 1 | 1 | 1 | 1 |
| A280 | 0.624 | 0.720 | 0.612 | 0.932 | 0.766 |
| Concentration | 2.17 mg/ml | 3.01 mg/ml | 2.54 mg/ml | 3.85 mg/ml | 3.16 mg/ml |
| Eluted protein volume | 20 ml | 20 ml | 20 ml | 20 ml | 20 ml |
| Protein level | 43.40 mg | 60.20 mg | 50.80 mg | 76.98 mg | 63.27 mg |

The expression levels of proteins were C3T16H3 > C3T16H4 > C3T16H1 > C3T16H2 > TE16c.

### (2) Concentration test on polished protein

An appropriate amount of sample was precisely measured, diluted with eluent by 10 to 50 folds, and stirred thoroughly with a glass rod. The absorbance was determined at 215 nm and 225 nm using an ultraviolet-visible spectrophotometry. The protein concentration was calculated based on the following formula: C (mg/ml) = (A215-A225) × 144 × dilution ratio ÷ 1000 (note: the absorbance value was required to be from 0.1 to 1).

The test results of the concentration were as follows:

| Plasmid | TE16c | C3T16H1 | C3T16H2 | C3T16H3 | C3T16H4 |
|---|---|---|---|---|---|
| Dilution ratio | 10 | 10 | 10 | 10 | 10 |
| A215 | 0.301 | 0.505 | 0.513 | 0.584 | 0.717 |
| A225 | 0.121 | 0.167 | 0.156 | 0.252 | 0.322 |
| Concentration | 0.26 mg/ml | 0.49 mg/ml | 0.51 mg/ml | 0.48 mg/ml | 0.57 mg/ml |
| Collected protein volume | 51 ml | 53 ml | 48 ml | 51 ml | 60 ml |
| Protein level | 13.26 mg | 25.80 mg | 24.68 mg | 24.38 mg | 34.13 mg |

The expression levels of proteins after polishing were C3T16H4 > C3T16H1 > C3T16H2 > C3T16H3 > TE16c.

### 8. Electrophoresis Test

The specific process was as follows: 40 µl of sample solution was measured. 10 µl of 5× protein loading buffer (250 mM Tris-HCl (pH 6.8), 10% (V/V) sodium dodecyl sulfate (SDS), 0.5% (V/V) bromophenol blue, 50% (V/V) glycerol, and 5% (V/V) β-mercaptoethanol) was added. The mixture was boiled in boiling water at 100°C for 10 min. Subsequently, the resulting solution was added to sodium dodecyl sulfate-polyacrylamide gel electrophoresis (SDS-PAGE) protein gel at 10 µl per well and subjected to electrophoresis at 80 V for 2 h. Thereafter, the protein was stained with Coomassie Brilliant Blue staining solution (0.1% (V/V) Coomassie Brilliant Blue R-250, 25% (V/V) isopropyl alcohol, and 10% (V/V) glacial acetic acid) for 20 min, and then decolorized with a protein decolorization solution (10% (V/V) acetic acid and 5% (V/V) ethanol).

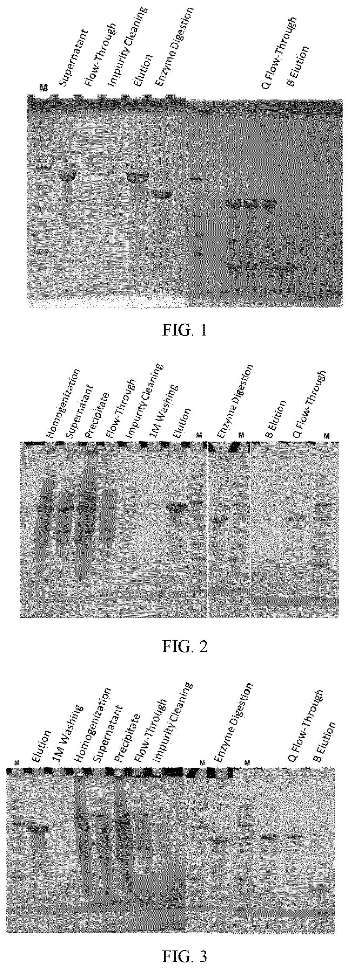

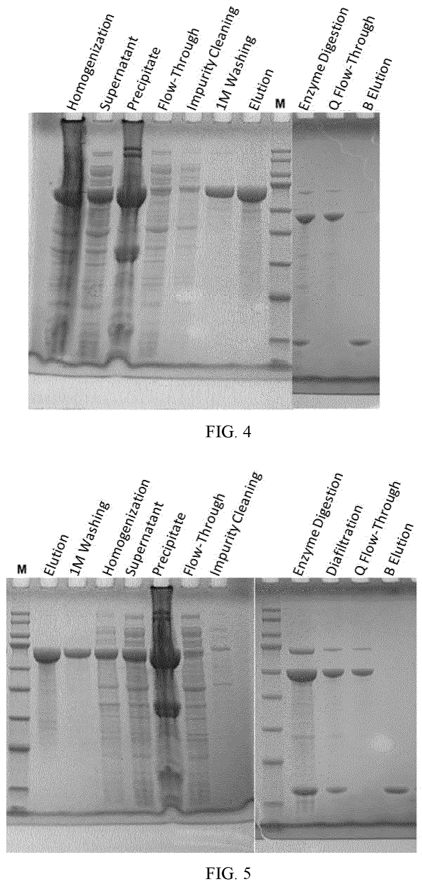

The electrophoresis test results were as shown in FIGS. 1 to 5. All the proteins C3T16H1, C3T16H2, C3T16H3, C3T16H4, and TE16c showed a good expression level, and the purified collagen had a better purity without obvious impurities.

### Example 2: Circular Dichroism Spectroscopy and UV Scanning Analysis of Recombinant Type III Humanized Collagen

### Experimental Method

(1) Setting of instrument parameters
   Band width: 1.0 nm;
   Step: 1.0 nm;
   Measurement range: 190-260 nm (far-UV region scan) / 250-340 nm (near-UV region scan);
   Time-per-point: 0.5 s;
   Repeats: 3 times;
   Cell Length: 10 mm|0.5 mm;
   Temperature: Room Temperature.
(2) Far/near-UV region scan of standard sample
   The scanning wavelength was set to 180-340 nm and the background test and the blank buffer test were carried out. Afterwards, the circular dichroism far- and near-UV absorption of 1 mg/mL CSA standard solution in the range of 180-340 nm was collected.
(3) Sample processing
   The C3T16H1, C3T16H2, C3T16H3, C3T16H4, and TE16c protein samples obtained after polishing were taken and concentrated to a protein concentration of 1 mg/ml in 10 kDa ultrafiltration concentrators (Millipore), respectively.
(4) Far-UV region scan of samples
   The cuvette was soaked in 2M HNO₃ overnight, rinsed with deionized water and air dried. The data of background was collected before the data of blank buffer was collected. Afterwards, an appropriate amount of the test sample was added to the cuvette to perform far-UV region scan at 190-260 nm according to the above parameters and the data were collected.
(5) Near-UV region scan of samples
   The cuvette was soaked in 2M HNO₃ overnight, rinsed with deionized water and air dried. The data of background was collected before the data of blank buffer was collected. Afterwards, an appropriate amount of the test sample was added to the cuvette to perform near-UV region scan at 250-340 nm according to the above parameters and the data were collected.
(6) Scanning spectra processing

All the spectra obtained after scanning were subjected to subtract baseline and smoothing by the software Pro-Data Viewer.

### Experimental Results and Analysis

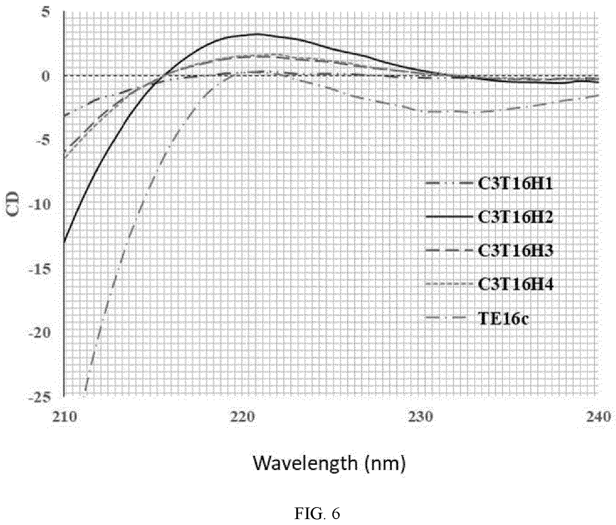

The experimental results were as shown in FIG. 6. All of C3T16H1, C3T16H2, C3T16H3, C3T16H4, and TE16c showed positive peaks at 221 nm, and the positive peak values were C3T16H2 > C3T16H4 ≥ C3T16H3 > C3T16H1 ≥ TE16c. This indicated that all of C3T16H1, C3T16H2, C3T16H3, C3T16H4, and TE16c had a triple helix structure under the same protein concentration conditions, the amino acid sequence in the hinge region of the human type III collagen contributed to formation of the triple helix structure of the recombinant type III humanized collagen, and the amino acid sequences of different lengths in hinge regions had different abilities to promote the recombinant type III humanized collagen to form a triple helix structure.

## Claims

1. A collagen self-assembled element, wherein the collagen self-assembled element comprises a sequence set forth in any one of the following (i) to (iii):
(i) at least 21 consecutive amino acids at C-terminus of the amino acid sequence set forth in SEQ ID NO.1;
(ii) an amino acid sequence obtained by substituting, deleting, or adding one or more amino acids in the amino acid sequence set forth in (i) and retaining a function of promoting self-crosslinking of a recombinant type III humanized collagen to form a triple helix structure; and
(iii) an amino acid sequence having at least 90%, 92%, 95%, 96%, 97%, 98%, or 99% identity to the amino acid sequence set forth in (i) and retaining the function of promoting the self-crosslinking of the recombinant type III humanized collagen to form the triple helix structure.

2. The collagen self-assembled element according to claim 1, wherein the sequence set forth in (i) is the amino acid sequence set forth in SEQ ID NO.1, SEQ ID NO.2, SEQ ID NO.3, or SEQ ID NO.4.

3. A use of the collagen self-assembled element according to claim 1 or 2 in promotion of the self-crosslinking of the recombinant type III humanized collagen to form the triple helix structure.

4. The use according to claim 3, wherein the collagen self-assembled element functions by being present at a terminus of an amino acid sequence of the recombinant type III humanized collagen;
preferably, the terminus of the amino acid sequence of the recombinant type III humanized collagen is C-terminus.

5. A recombinant type III humanized collagen, wherein the recombinant type III humanized collagen comprises the collagen self-assembled element according to claim 1 or 2 at a terminus thereof;
wherein preferably, the terminus of the recombinant type III humanized collagen is C-terminus.

6. The recombinant type III humanized collagen according to claim 5, wherein the recombinant type III humanized collagen comprises a sequence set forth in any one of the following (a) to (c):
(a) the amino acid sequence set forth in SEQ ID NO.6, SEQ ID NO.7, SEQ ID NO.8, or SEQ ID NO.9;
(b) an amino acid sequence obtained by substituting, deleting, or adding one or more amino acids in the amino acid sequence set forth in (i) and retaining an ability to self-crosslink and form the triple helix structure; and
(c) an amino acid sequence having at least 90%, 92%, 95%, 96%, 97%, 98%, or 99% identity to the amino acid sequence set forth in (i) and retaining the ability to self-crosslink and form the triple helix structure.

7. A polynucleotide, wherein the polynucleotide encodes the collagen self-assembled element according to claim 1 or 2, or encodes a recombinant type III humanized collagen according to claim 5 or 6;
preferably, the polynucleotide comprises a sequence set forth in the following (z₁) or (z₂):
(z₁) the nucleotide sequence set forth in SEQ ID NO.11, SEQ ID NO.12, SEQ ID NO.13, or SEQ ID NO.14;
(z₂) a nucleotide sequence that hybridizes with the nucleotide sequence set forth in (z₁) under stringency conditions and encodes a protein having an ability to self-crosslink and form the triple helix structure, wherein the stringency conditions are medium stringency conditions, medium-high stringency conditions, high stringency conditions, or very high stringency conditions.

8. A recombinant expression vector, wherein the recombinant expression vector comprises the polynucleotide according to claim 7.

9. A recombinant host cell, wherein the recombinant host cell comprises the recombinant expression vector according to claim 8.

10. A preparation method for the recombinant type III humanized collagen according to claim 5 or 6, wherein the preparation method comprises the following steps:
S1: constructing a recombinant expression vector comprising a polynucleotide according to claim 7 that encodes the recombinant type III humanized collagen according to claim 5 or 6, and constructing a recombinant host cell by transformation;
S2: culturing the recombinant host cell obtained in S1 in a medium and producing a protein;
S3: harvesting and purifying the protein, preferably purifying the protein by a Ni column and/or anion exchange chromatography; and
S4: optionally subjecting the protein to enzyme digestion, preferably subjecting the protein to the enzyme digestion with a tobacco etch virus (TEV) protease.
